# EUROPÄISCHE PATENTSCHRIFT

(11) **EP 3 771 453 B1**
(45) Veröffentlichungstag und Bekanntmachung des Hinweises auf die Patenterteilung: **04.03.2026**
(21) Anmeldenummer: 19189858.4
(22) Anmeldetag: 02.08.2019
(51) Int. Cl.: A61F 2/16

(54) **INTRAOKULARLINSE**
INTRAOCULAR LENS
LENTILLE INTRAOCULAIRE

(43) Veröffentlichungstag der Anmeldung: 03.02.2021
(73) Patentinhaber: L.O.O. GmbH, 45478 Mühlheim/Ruhr (DE)
(72) Erfinder: Ludwig, Udo, 13127 Berlin (DE); Tomalla, Mark, 45478 Mühlheim/Ruhr (DE)
(74) Vertreter: Schneider, Sascha

(56) Entgegenhaltungen:
- EP-A1- 3 476 375
- EP-A2- 1 155 665
- WO-A1-2019/098822
- DE-U1- 29 921 208
- FR-A1- 2 849 592
- FR-A1- 2 900 569
- FR-B1- 2 849 592
- FR-B1- 2 900 569
- US-A1- 2014 172 089
- US-A1- 2017 348 095
- US-A1- 2019 091 010
- US-A1- 2019 209 291
- US-B1- 6 986 787

## Beschreibung

Die Erfindung betrifft eine Intraokularlinse zum Einsetzen in das menschliche Auge. Herkömmliche Intraokularlinsen bestehen aus einer Optik und einem Fixationsgestell, die sogenannte Haptik.

Die Druckschriften DE 29921208, US 2019/091010 und FR 2900569 offenbaren Intraokularlinsen, die eine Optik und eine Vorspanneinrichtung umfassen, die als Spannring (2) ausgebildet wird, wobei der Spannring (2) eine radial nach außen gerichtete Haltekraft erzeugt.Die Duckschriften FR 2849592 und EP 1155665 beschreiben Vorspanneinrichtungen in Form von Spannringen, die in Kombination mit Intraokularlinsen mit mindestens einer Haptik verwendet werden sollen. In der Druckshrift US 6986787 wird eine Intraokularlinse beschrieben, die aus einer Optik und einer haptischen Struktur mit spiralförmiger Wicklung besteht.

Die Haptik hält die Optik in einer zentrierten Position und fixiert die Optik während des Heilungsprozesses des Kapselsackes. Während des Heilungsprozesses kommt es zu einer Kapselsackschrumpfung, welche den Federkräften der Haptik entgegenwirkt. Es können dadurch am hinteren Kapselsack Stressfalten entstehen, die optische Einflüsse ausüben können. Der gleiche Effekt entsteht, wenn der Kapselsack im Verhältnis zum Haptikdurchmesser zu klein ist.

Aufgrund des geringen Durchmessers der Optik kommt es nachteiligerweise dazu, dass der Patient bei Dunkelheit Blenderscheinungen hat, die durch Reflektionen am Optikrand entstehen. Zudem erschweren kleine Optiken den Einblick und das Arbeiten des Operateurs bei künftigen Behandlungen am Augenhintergrund (Netzhaut), da der Optikrand die Sicht des Operateurs behindert.

Es ist daher die Aufgabe der vorliegenden Erfindung die Nachteile des Stands der Technik zu beseitigen. Diese Aufgabe wird mit dem Gegenstand der unabhängigen Ansprüche gelöst. Vorteilhafte Weiterbildungen der Erfindung sind in den Unteransprüchen angegeben.

Die Erfindung betrifft eine Intraokularlinse aufweisend eine Optik, gemäß Anspruch 1.

Erfindungsgemäß weist die Intraokularlinse eine Vorspanneinrichtung auf. Vorteilhaft ermöglicht die Vorspanneinrichtung eine kontrollierte Entfaltung der Intraokularlinse im Kapselsack des Auges.

Vorteilhaft ist es dadurch möglich, Optiken mit einem Durchmesser größer oder gleich 8 mm, bevorzugt 9mm bis 14mm, in den Kapselsack des Auges einzusetzen. Dadurch ist es möglich eine Vollflächenoptik in das Auge einzusetzen. So kann der Kapselsack komplett durch die Intraokularlinse ausgefüllt werden, wobei der Kapselsack vollständig aufgespannt werden kann.

Vorteilhaft werden Blenderscheinungen reduziert. Zudem hat der Operateur bei Nachbehandlungen einen besseren Blick auf die Netzhaut. Durch den größeren Durchmesser der Optik liegt sie gleichmäßig auf der Rückseite des Kapselsacks auf. Dadurch werden Stressfalten reduziert.

Die Vorspanneinrichtung ermöglicht vorteilhaft eine gleichmäßige Ausspannung des Kapselsacks und eine verbesserte Zentrierung der Linse im Kapselsack.

Bevorzugt sind die Intraokularlinse und die Vorspanneinrichtung flexibel und/oder verformbar ausgebildet. Dadurch können sich vorteilhaft die Intraokularlinse und die Vorspanneinrichtung sehr gut an die Form des Auges bzw. des Kapselsacks anpassen. Zudem kann die Vorspanneinrichtung aufgrund der Flexibilität eine Haltekraft auf das Auges bzw. den Kapselsacks ausüben.

Bevorzugt ist die Vorspanneinrichtung aus demselben Material wie die Optik ausgebildet. Dadurch ist vorteilhaft eine besonders einfache Herstellung möglich, insbesondere können die Vorspanneinrichtung und die Optik einteilig ausgebildet sein.

Die Vorspanneinrichtung ist als Spannring ausgebildet. Dadurch kann vorteilhaft der Kapselsack gleichmäßig ausgespannt werden. Insbesondere wirkt der Spannring radial nach außen, sodass eine besonders gute Befestigung im Kapselsack möglich ist.

Bevorzugt ist die Vorspanneinrichtung in die Peripherie der Optik integriert. Dadurch ist vorteilhaft eine besonders gute Befestigung im Kapselsack möglich.

Die Optik weist an ihrer Peripherie eine, insbesondere sichelförmige, Öffnung zur Einbettung der Vorspanneinrichtung auf. Dadurch ist es vorteilhaft möglich, dass die Vorspanneinrichtung im Implantierten Zustand in der Öffnung angeordnet ist, wodurch ein besonders guter Sitz im Kapselsack möglich ist.

Bevorzugt weist die Intraokularlinse eine Öse für eine Injektionsvorrichtung bzw. eine Hakeneinrichtung einer Injektionsvorrichtung auf. Vorteilhaft kann durch die Öse die Intraokularlinse besonders präzise in den Kapselsack eingesetzt werden.

Bevorzugt ist die Intraokularlinse aufrollbar und/oder faltbar ausgebildet. So kann vorteilhaft die Intraokularlinse besonders präzise platziert werden.

Ein anderer Gegenstand der Offenlegung betrifft eine Injektionsvorrichtung zum Einsetzen der erfindungsgemäßen Intraokularlinse in das menschliche Auge. Die Injektionsvorrichtung verjüngt sich bevorzugt zur Spitze der Injektionsvorrichtung hin und ist bevorzugt konisch ausgebildet. Dadurch wird die Intraokularlinse vorteilhaft in Richtung der Spitze der Injektionsvorrichtung und damit in Injektionsrichtung eingerollt. Bevorzugt ist die Spitze abgerundet ausgebildet und passt in die Öse der Intraokularlinse.

Bevorzugt ist die Spitze zumindest teilweise angeraut, um Verwachsungen des Kapselsacks von der Optik zu lösen und diese breitflächig zirkular zu lösen. Die Injektionsvorrichtung weist bevorzugt ein Handstück auf, das bei der Implantation einhändig oder beidhändig bedienbar ist. Das Handstück ist bevorzugt formstabil ausgebildet.

Die Injektionsvorrichtung ist bevorzugt gebogen ausgebildet. Dadurch kann die Linse vorteilhaft besser ins Auge eingesetzt werden.

Bevorzugt ist die Injektionsvorrichtung transparent ausgebildet, wobei dadurch eine verbesserte Positionskontrolle der Intraokularlinse möglich ist.

Die Injektionsvorrichtung weist bevorzugt eine Führungsnut für die Vorspanneinrichtung der Intraokularlinse auf, wobei die Führungsnut bis zur Spitze verläuft. Dadurch kann vorteilhaft die Intraokularlinse besonders genau im Kapselsack justiert und zentriert werden. Die Führungsnut führt die Vorspanneinrichtung in Richtung der Spitze der Injektionsvorrichtung und somit in Injektionsrichtung.

Bevorzugt weist die Injektionsvorrichtung an der Spitze einen beweglichen Stempel zum Einsetzen der Intraokularlinse in den Kapselsack auf. Bevorzugt ist am Stempel eine Hakeneinrichtung angeordnet, wobei die Hakeneinrichtung zur Aufhängung der Intraokularlinse bzw. der Vorspanneinrichtung der Intraokularlinse ausgebildet ist. Bevorzugt ist die Hakeneinrichtung kippbar, um die Intraokularlinse bzw. die Vorspanneinrichtung der Intraokularlinse an der gewünschten Position auszuklinken. Danach kann die Injektionsvorrichtung aus dem Auge entfernt werden.

Weitere Vorteile, Merkmale und Einzelheiten der Erfindung ergeben sich aus der nachfolgenden Beschreibung bevorzugter Ausführungsbeispiele sowie anhand der Zeichnungen. Diese zeigen schematisch in:
- Fig. 1: zeigt eine erfindungsgemäße Intraokularlinse vor dem Einsetzen in das Auge,
- Fig. 2: zeigt die erfindungsgemäße Intraokularlinse nach dem Einsetzen in das Auge, und
- Fig. 3: zeigt eine Injektionsvorrichtung mit der erfindungsgemäßen Intraokularlinse.

In den Figuren sind gleiche Bauteile oder Bauteile mit der gleichen Funktion mit den gleichen Bezugszeichen gekennzeichnet.

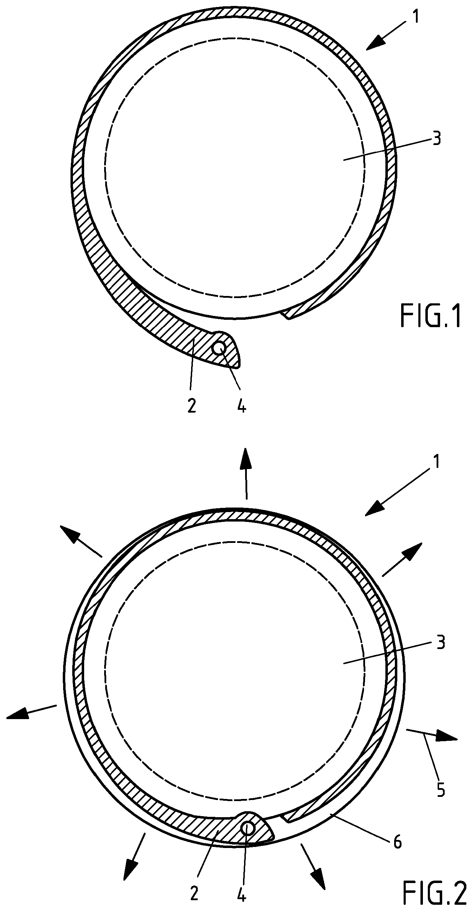

Fig. 1 zeigt eine erfindungsgemäße Intraokularlinse 1 vor dem Einsetzen in das Auge. Die Linse weist erfindungsgemäß eine Vorspanneinrichtung 2 auf, wobei die Vorspanneinrichtung 2 im entspannten Zustand ist. Im Inneren der Intraokularlinse 1 befindet sich eine Optik 3. Die Optik 3 hat einen Durchmesser größer oder gleich 8 mm, bevorzugt 9mm bis 14mm.

Die Vorspanneinrichtung 2 ist als Spannring ausgebildet und ist in die Peripherie der Optik 3 integriert und umgibt die Optik 3 an der Peripherie der Optik 3. Die Vorspanneinrichtung 2 ist über einen Umfangswinkel von ca. 270°-330° an der Optik 3 befestigt. Über den Restwinkel von ca. 90°-30° ist die Vorspanneinrichtung von der Optik 3 abgelöst. Zudem weist die Intraokularlinse 1 am abgelösten Teil der Vorspanneinrichtung eine Öse 4 auf, in die eine Injektionsvorrichtung 7 eingreifen kann.

Fig. 2 zeigt die erfindungsgemäße Intraokularlinse 1 nach dem Einsetzen in das Auge, sodass sich die Vorspanneinrichtung 2 im gespannten Zustand im Kapselsack 6 befindet. Die Vorspanneinrichtung 2 liegt eingebettet in einer Nut an der Peripherie der Optik 3. Durch die Elastizität der Vorspanneinrichtung 2 drückt die Vorspanneinrichtung 2 radial nach in Richtung der Kraftpfeile 5, sodass die Intraokularlinse 1 fest und präzise im Kapselsack 6 gehalten wird.

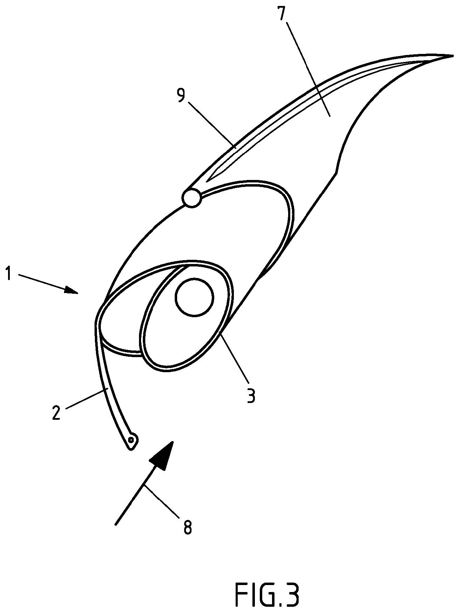

Fig. 3 zeigt eine Injektionsvorrichtung 7 mit der erfindungsgemäßen Intraokularlinse 1. Die Intraokularlinse 1 wird zusammengerollt in die hintere Öffnung der Injektionsvorrichtung 7 platziert. In Injektionsrichtung 8 verjüngt sich die Injektionsvorrichtung 7 zur Spitze hin, sodass die Spitze im Auge platziert werden kann. Die Vorspanneinrichtung 2 wird in einer zur Spitze verlaufenden Führungsnut 9 geführt, sodass die Intraokularlinse 1 von hinten nach vorne zur Spitze bewegt werden kann. Dabei wird die Intraokularlinse 1 immer weiter aufgerollt.

### Bezugszeichenliste

- 1: Intraokularlinse
- 2: Vorspanneinrichtung
- 3: Optik
- 4: Öse
- 5: Kraftpfeile
- 6: Kapselsack
- 7: Injektionsvorrichtung
- 8: Injektionsrichtung
- 9: Führungsnut

## Patentansprüche

1. Intraokularlinse (1) aufweisend eine Optik (3), wobei die Intraokularlinse (1) eine Vorspanneinrichtung (2) aufweist, die als Spannring (2) ausgebildet ist, wobei der Spannring (2) eine Haltekraft radial nach außen erzeugt, **dadurch gekennzeichnet dass** die Optik (3) an ihrer Peripherie eine Öffnung zur Einbettung des Spannrings (2) aufweist, wobei der Spannring (2) die Optik (3) an der Peripherie der Optik (3) umgibt.

2. Intraokularlinse (1) nach Anspruch 1, wobei die Optik (3) einen Durchmesser größer oder gleich 8 mm, bevorzugt 9mm bis 14mm, aufweist.

3. Intraokularlinse (1) nach einem der vorhergehenden Ansprüche, wobei die Intraokularlinse (1) und die Vorspanneinrichtung (2) flexibel und/oder verformbar, insbesondere aufrollbar und/oder faltbar, ausgebildet sind.

4. Intraokularlinse (1) nach einem der vorhergehenden Ansprüche, wobei die Vorspanneinrichtung (2) aus demselben Material wie die Optik (3) ausgebildet ist.

5. Intraokularlinse (1) nach einem der vorhergehenden Ansprüche, wobei die Vorspanneinrichtung (2) in die Peripherie der Optik (3) integriert ist.

6. Intraokularlinse (1) nach einem der vorhergehenden Ansprüche, wobei die Intraokularlinse (1) eine Öse (4) für eine Injektionsvorrichtung (7) aufweist.

## Claims

1. An intraocular lens (1) having an optic (3), wherein the intraocular lens (1) has a pre-tensioning device (2) which is constructed as a tension ring (2), wherein the tension ring (2) generates a radially outward retaining force, **characterized in that** the optic (3) has an opening at its periphery in order to embed the tension ring (2), wherein the tension ring (2) surrounds the optic (3) at the periphery of the optic (3).

2. The intraocular lens (1) according to claim 1, wherein the optic (3) has a diameter greater than or equal to 8 mm, preferably 9 mm to 14 mm.

3. The intraocular lens (1) according to one of the preceding claims, wherein the intraocular lens (1) and the pre-tensioning device (2) are flexible and/or deformable in construction, in particular rollable and/or foldable.

4. The intraocular lens (1) according to one of the preceding claims, wherein the pre-tensioning device (2) is constructed from the same material as the optic (3).

5. The intraocular lens (1) according to one of the preceding claims, wherein the pre-tensioning device (2) is integrated into the periphery of the optic (3).

6. The intraocular lens (1) according to one of the preceding claims, wherein the intraocular lens (1) has an eyelet (4) for an injection device (7).

## Revendications

1. Lentille intraoculaire (1) présentant une optique (3), la lentille intraoculaire (1) présentant un dispositif de précontrainte (2) conçu comme une bague de serrage (2), dans laquelle la bague de serrage (2) générant une force de maintien radialement vers l'extérieur, **caractérisée en ce que** l'optique (3) présente une ouverture pour l'incorporation de la bague de serrage (2) à sa périphérie, dans laquelle la bague de serrage (2) entoure l'optique (3) à la périphérie de l'optique (3).

2. Lentille intraoculaire (1) selon la revendication 1, dans laquelle l'optique (3) présente un diamètre supérieur ou égal à 8 mm, de préférence de 9 mm à 14 mm.

3. Lentille intraoculaire (1) selon l'une des revendications précédentes, dans laquelle la lentille intraoculaire (1) et le dispositif de précontrainte (2) sont conçu de manière flexible et/ou déformable, en particulier déroulable et/ou pliage.

4. Lentille intraoculaire (1) selon l'une des revendications précédentes, dans laquelle le dispositif de précontrainte (2) est conçu à partir du même matériau que l'optique (3).

5. Lentille intraoculaire (1) selon l'une des revendications précédentes, dans laquelle le dispositif de précontrainte (2) est intégré dans la périphérie de l'optique (3).

6. Lentille intraoculaire (1) selon l'une des revendications précédentes, dans laquelle la lentille intraoculaire (1) présente un œillet (4) pour un dispositif d'injection (7).
